Europäisches Patentamt

European Patent Office

Office européen des brevets

(11) Publication number: **0 597 454 A1**

## (12) EUROPEAN PATENT APPLICATION

(21) Application number: 93118174.7

(22) Date of filing: 10.11.93

(51) Int. Cl.5: **C07C 45/38**, C07C 47/127, B01J 23/50

(30) Priority: 12.11.92 JP 302269/92
11.12.92 JP 332000/92
17.12.92 JP 337308/92
18.12.92 JP 338585/92
18.12.92 JP 338586/92
23.03.93 JP 63603/93
21.06.93 JP 148888/93
28.06.93 JP 156420/93

(43) Date of publication of application:
18.05.94 Bulletin 94/20

(84) Designated Contracting States:
DE FR GB

(71) Applicant: MITSUI TOATSU CHEMICALS, Inc.
2-5 Kasumigaseki 3-chome
Chiyoda-Ku Tokyo 100(JP)

(72) Inventor: Sudoh, kazufuyu
2-2-1-25, Ayazono
Takaishi-shi, Osaka-fu(JP)
Inventor: Wakimura, Kazuo
31-230, Shindachiichiba
Sennan-shi, Osaka-fu(JP)
Inventor: Tanaka, Masao
1080-31, Fukainakamachi
Sakai-shi, Osaka-fu(JP)
Inventor: Miura, Toshio
3-9-517, Nishitoriishi
Takaishi-shi, Osaka-fu(JP)
Inventor: Inoue, Hatuo
482, Wada
Sakai-shi, Osaka-fu(JP)
Inventor: Yamazaki, Masato
2-5-212, Enokicho
Suita-shi, Osaka-fu(JP)

(74) Representative: Schüler, Horst, Dr.
Patentanwalt,
Kaiserstrasse 69
D-60329 Frankfurt (DE)

(54) **A method for preparing glyoxal and a catalyst for use in it.**

(57) There are here disclosed a method which comprises the step of oxdizing/dehydrogenating ethylene glycol at 500-700°C in the presence of a silver catalyst in which at least 50 wt% of silver is supported on a carrier or a silver catalyst having a melting point higher than the melting point of silver, a silver catalyst for use in this method, and a treatment method for activating the silver catalyst used in the oxidizing/dehydrogenating reaction. According to the utilization of these preparation methods and the catalysts, the sintering of the catalyst can be inhibited, the life of the catalyst can be maintained for a long time, and glyoxal can be prepared in a high yield, suppressing the production of by-products.

## BACKGROUND OF THE INVENTION

### (i) Field of the Invention

The present invention relates to a method for preparing glyoxal, and a catalyst for use in this method. More specifically, it relates to a method for preparing glyoxal by oxidizing/dehydrogenating ethylene glycol in the presence of a specific silver catalyst, and a catalyst for use in this method.

### (ii) Description of the Prior Art

Glyoxal is a very useful compound as a cross-linking agent acting as a hardener in various materials such as polymers, fibers, papers, cements and soils and an intermediate of organic synthesis.

Heretofore, as methods for preparing glyoxal, there are usually known methods which comprise oxidizing a corresponding alcohol compound, glycolaldehyde and the like, and among these methods, with regard to the techniques which comprise oxidizing/dehydrogenating ethylene glycol in the presence of a silver catalyst, many methods have been suggested.

For example, Japanese Patent Publication No. 54011/1986 discloses a method which comprises oxidation in the presence of a silver crystal having a certain particle diameter (0.1-2.5 mm). In Example 1 of this publication, reaction is carried out at a reaction temperature of 870°K, and the obtained results are conversion of 97.8%, glyoxal selectivity of 55% and a space-time yield of 14.6 g-glyoxal (GX)/cm$^3$-cat•hr. In this disclosed method, however, the conversion is low, and ethylene glycol, which is hardly separated from glyoxal, remains and contaminates the desired glyoxal. Therefore, the method is not satisfactory to industrially manufacture the high-quality product. U.S. Patent No. 4,555,583 (Japanese Patent Publication No. 49292/1990) discloses a method which comprises catalytically oxidizing ethylene glycol with a silver crystal in the presence of a vaporizable phosphorus compound. In Example 2 of this patent, reaction is carried out at a reaction temperature of 501°C, and the obtained results are ethylene glycol conversion of 100%, glyoxal selectivity of 80.4% and glycolaldehyde selectivity of 1.3%. However, this method also has the drawback that glycolaldehyde which is an impurity remains in large quantities, though the yield of the glyoxal is high. The production of glycolaldehyde can be decreased by the use of fine silver particles as a catalyst (Example 1), but the pressure loss of a catalyst layer increases unpreferably. Japanese Patent Application Laid-open No. 59933/1983 discloses a method which comprises catalytic oxidation with silver crystal containing phosphorus in the presence of a vaporizable phosphorus compound. In Example 1 of this application, ethylene glycol to which ammonium primary phosphate has been added is reacted in the presence of a catalyst formed by adding ammonium phosphate to a particulate silver catalyst, and in this case, a glyoxal yield of 80% is continued for 11 days, but a low space-time yield is disadvantageous.

Among these suggested techniques, there is an industrially practical method, which comprises oxidizing/dehydrogenating ethylene glycol in the presence of silver particles as a catalyst obtained by electrolysis. According to this method, the space-time yield is high, and a reaction liquid product can be obtained which contains small amounts of unreacted ethylene glycol and glycolaldehyde as an intermediate.

However, the silver particles obtained by this method are liable to be sintered at a high temperature. Therefore, if the reaction is continued in accordance with this method, the silver particles of the catalyst are mutually sintered, so that the pressure loss of the catalyst layer increases with time. As a result, a gas of a raw material or the like cannot be fed any more at a predetermined flow rate. In addition, when the reaction is carried out on an industrial scale, the catalyst layer with which a reactor is filled tends to contract, consolidate and conglomerate with time, and as this phenomenon proceeds, a gap appears between a catalyst bed and a wall of the reactor, and cracks inconveniently occur in the silver crystal layer on occasion.

Furthermore, there have also been suggested various methods for preparing dialdehyde which use a silver catalyst where silver is supported on a carrier, and in this case, the amount of silver supported on the carrier is as small as 5 to 30 wt%.

For example, in Izv. Akad. Nauk. SSSR, Ser. Khim., pp. 641-643 (1964), a catalyst in which silver is supported on alumina (the amount of supported Ag = 32 wt%) is used, and reaction is carried out at a reaction temperature of 600°C. In this case, however, the low results are merely obtained, and they are yield = 20% and space-time yield = 23.8 kg-GX/m$^3$-cat•hr. In Example 2 of Japanese Patent Publication No. 4816/1988, a catalyst in which silver is supported on alumina (the amount of supported Ag = 10 wt%) is used, and reaction is carried out at a reaction temperature of 400°C. In this case, the obtained results are conversion = 98%, selectivity = 65% and space-time yield = 120 kg-GX/m$^3$-cat•hr, but this space-time yield is unsatisfactory. In Example 3 of Japanese Patent Application Laid-open No.38227/1983, there is used a silver catalyst (the amount of supported Ag is 8 wt%) in which silver is supported on silicon carbide and which contains ammonium phosphate at the amount of 200 ppm in term of phosphorus, and in this case, the obtained results are ethylene glycol conversion = 97% and selectivity = 74%, but the space-time yield is as low as 0.26 kg-GX/kg-cat•hr. In Example 3 of Japanese Patent Application Laid-open No. 156739/1988, steatite spheres coated with silver are used as a catalyst, and reaction is carried out at a temperature of 360°C. In this case, the obtained results are conversion = 92.1%, selectivity = 67%, glycolaldehyde selectivity = 2.7% and space-time yield = 40 kg-GX/m$^3$-cat•hr. This method has the drawback that unreacted ethylene glycol and glycolaldehyde are large and the space-time yield is low, though the glyoxal selectivity is high. In addition, in Journal of Catalysis, Vol. 142, pp. 729-734 (1993), reaction is carried out at 550°C in the presence of a catalyst in which silver is supported on silicon carbide (the amount of supported Ag is 5 wt% or 8 wt%), and the results of conversion = 98.5%, selectivity = 73% and space-time yield = 920 kg-GX/m$^3$-cat•hr are obtained. However, there is the drawback that unreacted ethylene glycol remains in large quantities and the space-time yield is low.

The silver particles which can be used as the cata lyst are expensive, and therefore silver is reproduced from the used catalyst and then reutilized. For example, there are known a method in which used silver particles are dissolved in nitric acid, concentrated, and then recrystallized to obtain a silver nitrate crystal, and a silver nitrate solution prepared from this crystal is then electrolyzed to reproduce silver; and a method in which electrolysis is carried out in an electrolyte, while the used silver catalyst is directly utilized as an electrode, to precipitate silver particles. The reproduced silver has been industrially used as the catalyst. However, if such a silver catalyst which has been used for a long time, consolidated and conglomerated is repeatedly reproduced, the unreacted ethylene glycol and glycolaldehyde which is an impurity are accumulated, which is a serious problem for the preparation of the silver catalyst. Moreover, if a preparation lot is different or a preparation scale is different in the preparation of the supported silver catalyst, the amount of glycolaldehyde which is the impurity increases sometimes, even when ethylene glycol is reacted under the same conditions. In consequence, it has been difficult to prepare the catalyst having good reproducibility.

In the case that glyoxal is used as a fiber processing agent or a paper processing agent, a problem such as yellowing tends to occur unpreferably, if a large amount of impurities such as glycolaldehyde is present. Therefore, it is necessary to reduce these impurities as much as possible. Furthermore, it is difficult to separate and remove unreacted ethylene glycol and glycolaldehyde of a reaction intermediate from an aqueous glyoxal solution which is the product, and therefore the choice of severe preparing conditions and a proper catalyst is required so that the raw material ethylene glycol may not remain and glycolaldehyde of the reaction intermediate may not be produced in an oxidation/dehydrogenation process.

A metal such as silver, copper or its alloy as the catalyst is required to have a certain or more contact area in order to increase the yield of the reaction product. It should be noted that silver present in the vicinity of the surface of the catalyst is only effective as the silver catalyst for the reaction, and silver present inside the catalyst does not take part in the reaction and so it is vain. Therefore, the metal for the catalyst has been used in the form of shavings obtained by grinding a metal ingot with a lathe or the like, or in the form of a wire, a coil or a knitted wire net, but this working of the metal requires a huge cost.

As a solution of the problem of this working, it has been suggested to use, as the catalyst, a silver crystal produced by an electrolysis technique. However, in order to increase the yield of the reaction product, a certain or more contact area is essential, and for this purpose, silver particles are required to have a small diameter. However, the silver particles having the small diameter are gradually sintered in the reaction at a high temperature, so that it becomes difficult to feed the mixed reaction gases, and the operation of a plant becomes impossible with the increase of pressure loss. Even if not impossible, the decrease of the volume of the streaming gas leads to the noticeable drop of productivity. Alternatively, the silver particles having the small diameter tend to gradually contract, consolidate and conglomerate with time, so that a gap appears between a catalyst bed and a wall of the reactor, and cracks occur in the silver crystal layer inconveniently. Finally, an initial yield cannot be maintained any more in a short period of time, and the operation becomes impossible.

In addition, there are problems of the deterioration of the silver catalyst due to repeated use and reproduction, and the decline of catalytic power due to the above-mentioned cause regarding the preparation process.

## SUMMARY OF THE INVENTION

The present inventors have intensively and repeatedly investigated a method for preparing glyoxal by the gaseous phase oxidation of ethylene glycol in order to overcome the drawbacks of conventional methods, and as a result, the present invention has bee completed.

That is, the first aspect of the present invention is directed to a method for preparing glyoxal which comprises the step of oxidizing/dehydrogenating ethylene glycol at 500-700°C in the presence of a silver catalyst in which at least 50 wt% of silver is supported on a carrier.

The second aspect of the present invention is di rected to a method for preparing glyoxal which comprises the step of oxidizing/dehydrogenating ethylene glycol at 500-700°C in the presence of a catalyst comprising a silver alloy having a melting point higher than the melting point of silver.

The third aspect of the present invention is directed to a silver catalyst for the preparation of glyoxal in which at least 50 wt% of silver is supported on a carrier, and the fourth aspect of the present invention is directed to a silver catalyst for the preparation of glyoxal which comprises a silver alloy having a melting point higher than the melting point of silver.

The fifth aspect of the present invention is directed to a method for activating a silver catalyst for the preparation of glyoxal which comprises the step of immersing the silver catalyst used in the oxidizing/dehydrogenating reaction of ethylene glycol into an acid which does not react with silver.

The feature of the first invention resides in that the carrier for the catalyst is used and the amount of supported silver is regulated so as to be 50 wt% or more based on the total weight of the catalyst. It falls off from the conception of conventional ordinary techniques to regulate the amount of the supported silver to 50 wt% or more, and this high silver content can prevent the conventional sintering of the silver catalyst particles, and can secure the high conversion of ethylene glycol and the high selectivity of glyoxal, while maintaining the life of the catalyst for a long time, which is surprising in view of the conventional techniques.

## DETAILED DESCRIPTION OF THE PREFERRED EMBODIMENTS

No particular restriction is put on a carrier for a catalyst which can be used in the present invention, so long as it can be used as a carrier for a usual catalyst, but the preferably usable carrier has little activity as the catalyst in itself, a relatively small specific surface area and a relatively small particle diameter.

Examples of the carrier include oxides, nitrides, carbides and their intermediate compositions, simple metals and alloys, and other inactive inorganic compounds.

Of these examples, there can be preferably used, for example, silica having a specific surface area of 5 $m^2/g$ or less, preferably 0.01 to 2.0 $m^2/g$ and a particle diameter of 10 to 1,000 $\mu m$, prefer-

ably 40 to 500 $\mu m$; $\alpha$-alumina having a specific surface area of 5 $m^2/g$ or less, preferably 0.03 to 2.0 $m^2/g$ and a particle diameter of 10 to 500 $\mu m$, preferably 25 to 500 $\mu m$; or an oxide (except the above-mentioned silica and $\alpha$-alumina), a nitride, a carbide or its intermediate composition having a specific surface area of 10 $m^2/g$ or less, preferably 0.01 to 5.0 $m^2/g$ and a particle diameter of 10 to 500 $\mu m$, preferably 25 to 500 $\mu m$.

If silver is supported on the carrier having the high specific surface area, reaction is unstable and so the reaction cannot be stably continued for a long period, and the production of impurities such as organic acids, carbon monoxide and carbon dioxide increase unpreferably. On the other hand, if the carrier has the large particle diameter, the contact area of the carrier which comes in contact with a reaction gas is small, so that it is unpreferably necessary to increase the amount of the catalyst to be filled.

Furthermore, if an oxide, a nitride or a carbide such as titanium oxide or silicon carbide or its intermediate composition having high dielectric constant is used as the carrier, the wettability of the carrier to silver can be further improved, the carrier can be uniformly coated with silver, the life of the catalyst can be prolonged, and the amount of silver to be used can be reduced, though the reason for these advantages is indefinite. In this case, the substance having a dielectric constant of 25 or more is preferably used.

The silver catalyst of the present invention can be prepared by first mixing a catalyst carrier such as the above-mentioned silica or alumina with a silver salt solution, drying the mixture, and then subjecting the dried mixture to a heat treatment to thermally decompose the silver salt, or by supporting silver on the carrier by plating or the like.

As the silver salt solution, there can be used an aqueous solution, an alcohol solution or their mixed solution of silver salts such as silver nitrate and silver lactate, or a complex ion solution of ammonia or the like and silver. The silver catalyst obtained by mixing the silver salt solution and the catalyst carrier can be prepared by, for example, suspending the catalyst carrier in the silver salt solution, followed by filtration, or by slowly adding the silver salt solution to the catalyst carrier so that the silver salt solution may not ooze out, drying, heating, and then reducing the mixture. In general, for the purpose of increasing the amount of supported silver, the serial treating operation of mixing the silver salt solution with the catalyst carrier, drying, heating, reducing, and then milling the mixture is repeated several times.

The thus prepared silver catalyst is subjected to analysis by the use of SEM (scanning electron microscope) or XMA (X-ray microanalyzer), and it is confirmed that the silver catalyst scarcely coheres and the surface of the carrier is coated with metallic silver.

The amount of silver in the silver catalyst supported on the carrier consisting of the oxide, the nitride, the carbide or the intermediate composition thereof which can be used in the present invention is 50 wt% or more, preferably 50 to 95 wt%, more preferably 50 to 90 wt%, based on the total weight of the finally completed catalyst. Since silver has much larger specific gravity than the carrier to be used, the amount of silver as much as 90 wt% is such that the surface of the carrier is just completely covered. Even if the amount of the supported silver is excessively increased, the present invention can be achieved, but the production of glyoxal does not increase so much and the number of the repeating operation of the mixing of the silver salt solution and the catalyst carrier, the drying and the heating is increased, which makes the process intricate. Conversely, if the amount of the supported silver is less than 50 wt%, the reaction is carried out at a low reaction temperature of from 300 to 500°C at low catalyst load in order to raise selectivity. In this case, conversion lowers and so the yield of glyoxal decreases, though the selectivity of glyoxal can be raised. If the catalyst load is increased in order to heighten the yield, the amounts of unreacted ethylene glycol and glycolaldehyde increase. Furthermore, if the reaction temperature is raised, the production of CO and $CO_2$ increases and the production of glyoxal decreases unpreferably, and the sintering of silver proceeds and the reaction becomes unstable and cannot be continued any more.

A metal which can be used in the silver alloy cata lyst of the present invention must form a silver alloy which has a melting point higher than the melting point (about 960°C) of silver, and the metal to be added may be one kind of metal, two or more kinds of metals, or an alloy.

In the present invention, the melting point of the silver alloy catalyst is raised, whereby the conversion of ethylene glycol and the selectivity of glyoxal in the case of a conventional method can be secured, the sintering of the silver catalyst particles can be prevented, and the life of the catalyst can be maintained for a long period. Examples of the metal which can be added to silver in order to form the silver alloy catalyst for use in the present invention include gold, platinum, palladium and so on. The melting point of the silver alloy with this kind of metal depends upon the amount of the metal to be added, and if the melting point of the silver alloy is raised by adding a certain amount of the metal, the silver alloy is scarcely sintered, even when ethylene glycol is oxidized/dehydrogenated at a reaction temperature of from 500 to 700°C.

No particular restriction is put on the content of silver, but the content of silver is preferably 50 wt% or more, that is, the content of the metal component which can provide the silver alloy with a melting point higher than the melting point of silver is preferably 50 wt% or less. If the content of silver is excessively low, the conversion of ethylene glycol uneconomically lowers, and a catalyst cost unreasonably increases.

As a method for preparing the silver alloy catalyst of the present invention, various methods can be utilized. For example, there are a method which comprises mixing silver with an added metal, melting the metal mixture, solidifying the molten mixture to obtain a solid, and then working the solid into a wire or shavings, and a method which comprises spraying/quenching the molten mixture to form particles. The thus obtained silver alloy having the optional form can be used as the catalyst. Moreover, the silver alloy which is supported on catalyst carriers such as alumina, silica, titania and silicon carbide can be also used as the catalyst.

Next, reference will be made to a supported silver catalyst obtained by plating which can be used in the present invention. This supported silver catalyst can usually and economically be prepared by electroplating, and in this case, a simple metal or an alloy having an optional shape is used as a cathode in a solution in which silver ions are complexed with optional complexing agents such as cyanide and ammonia. Furthermore, the particles or the film of silver formed on the surface of the simple metal or the alloy by chemical plating, vapor deposition, ion plating or the like can also be used as the catalyst. No particular restriction is put on the thickness of the silver plating, but it is suitably in the range of from 1 to 100 $\mu$m and regulation is made so that the amount of silver may be 50 wt% or more based on the total weight of the finally completed catalyst.

Examples of the carrier on which silver can be plated or vapor-deposited include simple metals such as copper and nickel as well as alloys such as brass and bronze. The usable carrier may take a form of wire, wire net, woven metallic fiber, gauze or cottony metal, and it is now industrially manufactured. In short, the carrier which is usually and widely used in many fields can be used. For example, the wire net of a sieve or a filter which is used for the purpose of classification or filtration can be used. One advantage of using this material is, needless to say, that it is inexpensive. In addition, this kind of carrier material can be molded into a thin wire shape or a fibrous shape into which simple silver cannot be molded, and thus the carrier has a large contact area, which permits the amount of silver to be reduced.

As particulate inorganic compounds which can be used in a silver-plated inorganic particulate catalyst, there can be used $\alpha$-alumina, zirconia, silicon carbide and silicon nitride which scarcely have catalytic activity in itself and which have a specific surface area of 5 $m^2$/g or less.

The supported silver catalyst obtained by silver plating which can be used in the present invention can be prepared by forming silver particles or a silver film on the surfaces of the inorganic particles by electroplating or chemical plating. Moreover, in order to improve the adhesive strength between the inorganic particles and silver, the following method can be utilized.

That is, since the inorganic particles are an insulator, the silver plating which is excellent in adhesive properties cannot be formed by the usual electroplating. Therefore, the surfaces of the inorganic particles are first chemically plated with Pd, Ag, Cu or Ni to make them conductive, and the electroplating or the chemical plating is then carried out with silver to obtain the silver-plated catalyst having the good adhesive strength. Alternatively, the inorganic particles can be coated with silver by techniques such as vapor deposition and ion plating to prepare the usable silver catalyst.

The thickness of the silver film plated on the inorganic particles is usually from 0.1 to 50 $\mu$m, preferably from 1.0 to 20 $\mu$m, and regulation is preferably made so that the amount of silver may be 50 wt% or more, preferably 50 to 95 wt%, more preferably 50 to 90 wt% based on the total weight of the finally completed catalyst.

The feature of the silver-plated catalyst prepared by this method resides in that heat resistance is higher than the silver crystal catalyst having a similar particle diameter and consolidation and conglomeration are inhibited, when used at a high temperature. In general, an oxide is less sintered than a metal, and accordingly, the reason why the silver-plated catalyst is scarcely sintered would be that an occupation ratio of the silver oxide on the surface of the silver-plated catalyst is higher than that of the metal silver, though this reason is not definite.

The silver-plated catalyst has the high heat resistance, and so there scarcely exist consolidation and conglomeration which tend to occur in the case that the silver crystal is employed, and the increase of pressure loss is prevented. Therefore, the silver-plated catalyst can possess a smaller particle diameter, and even if the amount of the catalyst is decreased, the contact area of the catalyst can be advantageously maintained.

Furthermore, the silver-plated catalyst can be used together with the silver crystal catalyst. For example, a reactor is filled with the silver-plated catalyst so that the same may be put on the silver crystal layer, or conversely, the reactor is filled with the silver crystal layer so that the same may be put on the silver-plated catalyst. Alternatively, the silver-plated catalyst may be mixed with the silver crystal, whereby the consolidation and conglomeration occur less than when the silver crystal catalyst is singly used.

In the present invention, silver can be supported on the particles of a simple metal or an alloy. When the carrier of the metal or the alloy has an excessively large particle diameter, the contact area of the catalyst with the reaction gas decreases, so that the amount of the catalyst to be supported must be unpreferably increased. Conversely, when the particle diameter is excessively small, the particles finally cohere into large grains in a step in which silver is supported. In consequence, the particle size of the metal or the alloy is usually in the range of from 10 to 500 $\mu$m, preferably from 25 to 500 $\mu$m.

As the particles of the metal or the alloy which is used as the catalyst carrier, there can be used electrolyzed copper particles obtained by a spray/quench method, or other particles. As a technique of supporting silver on the sur faces of the simple metal or the alloy, there can be used an immersion method using a solution of silver salts such as silver nitrate and silver lactate, or plating methods such as electroplating and chemical plating.

The amount of silver to be supported on the particles of the metal or the alloy is 50 wt% or more, preferably 50 to 90 wt% based on the total weight of the finally completed catalyst. Even if the amount of silver to be supported is largely increased, the production of glyoxal does not increase so much, though the present invention can be achieved. If the amount of the silver to be supported is small, the production of glyoxal decreases, and in order to heighten the yield, if the reaction temperature is raised, the reaction becomes unstable and cannot be continued any more.

The supported silver catalyst prepared by steps of the immersion of the carrier into the silver salt solution, drying, calcining, milling and the like, the supported silver catalyst prepared by the plating method, the catalyst of the silver alloy, or the silver catalyst of the silver crystal obtained by the electrolysis can be treated with an acid which does not react with silver to remove impurities from the active surface of the catalyst, thereby further improving catalytic performance. Examples of the acid include hydrochloric acid and acetic acid. After the acid treatment, the catalyst can be further treated with an alkali to remove the impurities. Examples of the usable alkali include aqueous solutions of ammonia, hydroxides, carbonates and hydrogencarbonates of alkali metals, and hydroxides, carbonates and hydrogencarbonates of alkaline earth metals. The acid treatment can be achieved by immersing the catalyst particles in the acid solution, or by passing the acid solution through the layer of the catalyst particles with which a reactor is filled. However, no particular restriction is put on the procedure of the acid treatment, so long as it can remove the impurities from the catalyst with the aid of the acid. Additionally, after the acid treatment or after the alkali treatment, the catalyst particles may be washed with water. When the treatment is carried out with ammonia water, water washing can be omitted.

The impurities of the supported silver catalyst or the silver-plated catalyst are substances which contaminate the active surface of the catalyst mainly in operations such as electrolysis, crushing, milling and classification. Examples of the impurities include Fe, Ni, Cr and Si. If the amount of the impurities is reduced to 10 ppm by these treatment, the performance of the catalyst can be further improved.

If the amount of the impurities contained in the silver catalyst increases, the leakage of ethylene glycol which is the raw material increases and the amount of glycolaldehyde which is the intermediate of the reaction also increases, as compared with the silver catalyst containing a less amount of the impurities, ever under the same operating conditions. In order to decrease the amounts of the unreacted raw material and the reaction intermediate, if the oxidizing/dehydrogenating reaction is carried out at a high temperature, the yield of glyoxal unpreferably lowers.

The silver catalyst of the present invention is scarcely sintered, even when used in the oxidizing/dehydrogenating reaction of ethylene glycol, and so it can be easily reutilized. That is, the used catalyst layer can be easily reutilized only by lightly crushing and then classifying the same. Alternatively, the used catalyst can be calcined in air in order to remove organic substances and carbon which adhere to the surface of the catalyst, or it can be immersed in the above-mentioned acid which does not react with silver, thereby reproducing the catalyst. In addition, the immersion in the silver salt solution, drying, calcination and classification may be added, whereby a more stable catalytic performance can be obtained.

When the catalyst of the present invention is subjected to the above-mentioned activation treatment, the activity of the catalyst can be improved, so that the amounts of the unreacted ethylene glycol and glycolaldehyde can be reduced, whereby glyoxal can be prepared with good reproducibility.

The features of the supported silver catalysts prepared by these methods are the high heat resistance and less consolidation, conglomeration and sintering during use at a high temperature, though its reason is not definite. According to surface analysis by ESCA (Electron Spectoroscopy for Chemical Analysis), it is apparent that oxidized silver is present on the surface of the supported silver catalyst which has undergone the oxidizing/dehydrogenating reaction of ethylene glycol. Furthermore, according to observation by SEM image, it is apparent that silver is piled up and polycrystallized on the surface of the carrier, and many grain boundaries are observed. It can be considered that the formation of these grain boundaries and the formation of the silver oxide film prevent the sintering, i.e., the growth of the silver particles.

In the case of the catalyst on which a small amount of silver is supported, the activity promptly lowers, when the reaction is continued. Observing the used catalyst in this case by SEM, it is apparant that the exposed portions of the carrier have increased and the silver particles have grown in a spherical form on the surface of the carrier. Thus, when the amount of the supported silver is insufficient, it can be considered from these results that the surface area of silver effective for the reaction decreases, which leads to the deterioration of the activity.

If the reaction is continued using electrolyzes silver which is pure silver as the catalyst, the pressure loss of a catalyst layer increases. Since being a substantially simple crystal, the electrolyzed silver is considered to be more easily sintered, as compared with the catalyst of the present invention which comprises polycrystalline silver.

In the present invention, molecular oxygen is used in the oxidizing/dehydrogenating reaction of ethylene glycol. As the molecular oxygen, pure oxygen or air may be used, but from an economical viewpoint, the latter is better. In order to obtain glyoxal in a high yield, a method can be used in which ethylene glycol and the molecular oxygen are diluted with an inert gas and the reaction is then carried out. Examples of the usable inert gas include nitrogen, rare gases such as helium and argon, a carbon dioxide gas and steam.

In the gaseous phase oxidation of the method of the present invention, a suitable reaction temperature is in the range of from 500 to 700°C, preferably from 530 to 660°C. If the reaction temperature is lower than 500°C, the unreacted ethylene glycol and the production of glycolaldehyde increase. Conversely, if the reaction temperature is higher than 700°C, the productions of formaldehyde, carbon monoxide and carbon dioxide increase, so that the production of glyoxal unpreferably decreases.

In the present invention, it is preferable that the oxidizing/dehydrogenating reaction of ethylene glycol is carried out in the presence of phosphorus or a phosphorus compound, because the yield of glyoxal can be improved. The amount of phosphorus or the phosphorus compound to be added is preferably in the range of from 0.01 to 10.0 ppm in term of phosphorus to ethylene glycol. A predetermined amount of phosphorus or the phosphorus compound may be mixed with ethylene glycol and then fed to the reaction, or alternatively, phosphorus or the phosphorus compound may be added independently singly or in the form of a solution to the reaction system. Examples of the effectively usable phosphorus compound include primary to tertiary phosphines such as mono, di and trimethylphosphines, phosphites such as methyl phosphite and ethyl phosphite, and various organic phosphorus compounds such as methylphosphonic acid dimethyl ester and ethylphosphonic acid diethyl ester.

However, the phosphorus compound having a high boiling point is not preferable, because the temperature of a vaporizer must be particularly raised, or because the phosphorus compound stagnates, decomposes and accumulates in the vaporizer, so that a material of the vaporizer is corroded and the resultant iron rust pieces might get into a reaction layer to badly affect the reaction. In consequence, more preferably, there can be used organic phosphorus compounds having relatively low boiling points, for example, methyl phosphite, ethyl phosphite, methyl phosphate and ethyl phosphate.

When phosphorus or the phosphorus compound is added, the production of oxidants such as carbon monoxide and carbon dioxide and decomposition products such as formaldehyde can be remarkably inhibited in contrast to a case where phosphorus or the phosphorus compound is not added, so that the yield of glyoxal which is the desired product is noticeably improved.

When the catalyst of the present invention is used, a space-time yield can be remarkably increased, as compared with the space-time yield (0.26-920 kg of glyoxal/$m^3$-cat•hr) in the case of a conventional supported silver catalyst, and even under such severe conditions as reduce the amounts of ethylene glycol and glycolaldehyde, there can be obtained the space-time yield (thousands kg-GX/$m^3$-cat•hr or more) equal to or more than the case of an electrolyzed silver catalyst.

The life of the catalyst in the present invention means the deterioration of a usual catalytic activity and is also concerned with the rise of pressure loss attributed to the sintering of the catalyst layer with the continuation of the reaction. That is, the latter meaning of the catalyst life is that when glyoxal is produced on an industrial scale, the reaction gas does not flow at a predetermined flow rate with the rise of the pressure loss. The decrease of the gas feed directly leads to the deterioration of productivity, and it indirectly leads to that a residence time is prolonged and glycolaldehyde and the like are increased in the subsequent reaction.

The pressure loss of the catalyst layer usually increases with the progress of the reaction. This pressure loss which rises with time is inversely proportional to the life of the catalyst. For example, when a certain catalyst is used for a same reaction time as a different catalyst used and the pressure loss of the catalyst layer increases twice of the different catalyst layer, the life of the catalyst corresponds to substantially half of that of the different catalyst. The life of the catalyst, particularly the supported silver catalyst of the present invention is twice or three times as long as that of the conventional silver catalyst, whereby the reaction can be kept up for a longer time than when the conventional catalyst is used. Furthermore, according to the method which comprises oxidizing/dehydrogenating ethylene glycol by the use of the catalyst of the present invention, glyoxal can be prepared in a high yield, the production of the by-products being inhibited, and it is definite that the present invention is industrially very advantageous.

Now, the present invention will be described in detail in reference to examples, but the scope of the present invention should not be limited to these examples.

Example 1

100 g of silica having a specific surface area of 0.09 $m^2$/g and a particle size distribution of 10 wt% of 400-250 $\mu$m, 15 wt% of 250-150 $\mu$m, 29 wt% of 150-105 $\mu$m, 33 wt% of 105-75 $\mu$m, and a balance of 75-25 $\mu$m were impregnated with 50 g of a silver nitrate solution (which was prepared from 200 g of silver nitrate and 100 g of water), and the silica particles were mixed arid then dried at 100°C with mixing. The thus dried particles were subjected to a heat treatment at 600°C for 30 minutes in the atmosphere, cooled, and then milled. Afterward, the same impregnation, drying, heat treatment and milling were repeated several times to prepare a catalyst containing 88 wt% of supported silver. Next, the obtained supported silver catalyst was classified, and a stainless steel reactor having an inner diameter of 27.4 mm was then filled with 5 ml of the obtained catalyst having particle sizes of 500-250 $\mu$m as the lowermost layer, 4 ml of the catalyst having particle sizes of 250-150 $\mu$m as the middle layer and 3 ml of the catalyst having particle sizes of 150-105 $\mu$m as the uppermost layer.

Into the thus-prepared reactor, ethylene glycol at 300 g/hr added trimethyl phosphite at the amount of 1 ppm as phosphorus to the ethylene glycol, water at 300 g/hr, air at 570 liters/hr and nitrogen at 1,300 liters/hr were charged through a vaporizer and a preheater, as a downward flow. They were reacted at a reaction temperature of 620°C for one day. After cooling the reaction gas, reaction products were separated and collected in an absorption tower which employed water as its absorbent. In this case, the pressure loss of catalyst layers was 0.35 kg/cm$^2$.

The results on the first day after the start of the reaction were that the conversion of ethylene glycol was 98.0%, the selectivity of glyoxal was 62.5%, the selectivity of formaldehyde was 12.6%, and the selectivity of glycolaldehyde was 0.46%. A space-time yield was 14.3 g-GX/cm$^3$-cat·hr.

The feeds of 300 g/hr of ethylene glycol, 300 g/hr of water, 570 liters/hr of air and trimethyl phosphite at the amount of 1 ppm as phosphorus to ethylene glycol were kept constant, and the feed of nitrogen was controlled so that the reaction temperature might be 620°C. Thirty days after the start of the reaction, the feed of nitrogen which was used to maintain the reaction temperature at 620°C was the same as on the first day, 1,300 liters/hr, and the pressure loss of the catalyst layers was 0.36 kg/cm$^2$.

The oxidizing/dehydrogenating reaction of ethylene glycol in the present invention was an exothermic reaction, and the control of the temperature was carried out by the introduction of nitrogen. Thus, judging from the fact that the feed of nitrogen was the same as on the first day, the reaction was stable, and therefore it could be considered that the activity of the catalyst scarcely changed from the initial stage.

The results of the reaction were that the conversion of ethylene glycol was 97.8%, the selectivity of glyoxal was 52.8%, the selectivity of formaldehyde was 11.2%, and the selectivity of glycolaldehyde was 0.39%. The pressure loss of the catalyst layers and the results of the reaction scarcely changed from the initial results.

Comparative Example 1

A stainless steel reactor having an inner diameter of 27.4 mm was filled with silver particles obtained by a usual electrolysis process at 5 ml of 500-250 $\mu$m as the lower layer, at 5 ml of 250-150 $\mu$m as the middle layer and at 5 ml of 150-105 $\mu$m as the upper layer.

Into the thus-prepared reactor, ethylene glycol at 125 g/hr added trimethyl phosphite at the amount of 3.0 ppm as phosphorus to the ethylene glycol, water at 125 g/hr, air at 235 liters/hr and nitrogen at 400 liters/hr were charged through a vaporizer and a preheater, as a downward flow. They were reacted at a reaction temperature of 575°C. The resultant reaction gas was cooled, and reaction products were separated and collected in an absorption tower which employed water as its absorbent. In this case, the pressure loss of catalyst layers was 0.16 kg/cm$^2$. The results of the reaction were that the conversion of ethylene glycol was 99.9%, the selectivity of glyoxal was 60.7%, the selectivity of formaldehyde was 8.6%, and the selectivity of glycolaldehyde was 0.36%. A space-time yield was 4.7 g-GX/cm$^3$-cat·hr.

Ethylene glycol, water, air and trimethyl phosphite were added into the reactor at 125 g/hr, 125 g/hr, 235 liters/hr and 3.0 ppm in term of phosphorus to ethylene glycol, respectively, and these conditions were kept constant and the feed of nitrogen was controlled so that the reaction temperature might be 575°C. Fifteen days after the start of the reaction, the feed of nitrogen was the same as on the first day, 400 liters/hr, which meant that a catalyst activity was the same as on the first day. Thirty days after the start of the reaction, the feed of nitrogen was 390 liters/hr and the pressure loss of the catalyst layers was 0.33 kg/cm$^2$. Forty-five days after the start of the reaction, the feed of nitrogen was 380 liters/hr and the pressure loss of the catalyst layers was 0.41 kg/cm$^2$.

The results of the reaction were that the conversion of ethylene glycol was 99.8%, the selectivity of glyoxal was 59.9%, the selectivity of formaldehyde was 8.2%, and the selectivity of glycolaldehyde was 0.45%.

The results of the reaction scarcely changed from those of the initial reaction, but the pressure loss of the catalyst layers increased. If the pressure loss rises in this way at the time of the preparation of glyoxal on an industrial scale, it is difficult to feed the gases at predetermined flow rates, so that the feeds of the gases decrease, in other words, the productivity of glyoxal unpreferably decreases.

Example 2

100 g of silica having a specific surface area of 82 m$^2$/g and particle sizes of 350-75 $\mu$m were impregnated with 50 g of a silver nitrate solution (which was prepared from 200 g of silver nitrate and 100 g of water), and the silica particles were mixed and then dried at 100°C with mixing. The thus dried particles were subjected to a heat treatment at 600°C for 30 minutes in the atmosphere, cooled, and then milled. Afterward, the same impregnation, drying, heat treatment and milling were repeated several times to prepare a catalyst containing 51 wt% of supported silver. Next, the obtained supported silver catalyst was classified, and a stainless steel reactor having an inner diameter of 27.4 mm was then filled with 10 ml of the obtained catalyst having particle sizes of 500-250 $\mu$m as the lowermost layer, 10 ml of the catalyst having particle sizes of 250-150 $\mu$m as the middle layer and 10 ml of the catalyst having particle sizes of 150-105 $\mu$m as the uppermost layer.

Into the thus-prepared reactor, ethylene glycol at 136 g/hr added trimethyl phosphite at the amount of 0.5 ppm as phosphorus to the ethylene glycol, water at 136 g/hr, air at 270 liters/hr and nitrogen at 700 liters/hr were charged through a vaporizer and a preheater, as a downward flow. They were reacted at a reaction temperature of 620°C for one day. After cooling the reaction gas, reaction products were separated and collected in an absorption tower which employed water as its absorbent. In this case, the pressure loss of catalyst layers was 0.30 kg/cm$^2$.

The results on the first day after the start of the reaction were that the conversion of ethylene glycol was 99.2%, the selectivity of glyoxal was 46.3%, the selectivity of formaldehyde was 13.4%, and the selectivity of glycolaldehyde was 0.42%. A space-time yield was 2.0 g-GX/cm$^3$-cat•hr.

Furthermore, the feed of nitrogen was controlled to maintain the temperature at 620°C, and 10 days after the start of the reaction, the feed of nitrogen was 470 liters/hr, the pressure loss of the catalyst layers was 0.21 kg/cm$^2$, and the reaction was slightly unstable.

The results of the reaction were that the conversion of ethylene glycol was 97.5%, the selectivity of glyoxal was 42.8%, the selectivity of formaldehyde was 12.5%, and the selectivity of glycolaldehyde was 0.76%.

Example 3

Silica having a specific surface area of 82 m$^2$/g and particle sizes of 350-75 $\mu$m was subjected to a heat treatment at 1,100°C for one hour to obtain a catalyst carrier having a specific surface area of 0.2 m$^2$/g. The same procedure as in Example 1 was carried out to prepare a catalyst containing 52 wt% of supported silver. Next, the obtained supported silver catalyst was classified, and a stainless steel reactor having an inner diameter of 27.4 mm was then filled with 10 ml of the obtained catalyst having particle sizes of 500-250 $\mu$m as the lowermost layer, 10 ml of the catalyst having particle sizes of 250-150 $\mu$m as the middle layer and 10 ml of the catalyst having particle sizes of 150-105 $\mu$m as the uppermost layer.

Into the thus-prepared reactor, ethylene glycol at 136 g/hr added trimethyl phosphite at the amount of 0.5 ppm as phosphorus to the ethylene glycol, water at 136 g/hr, air at 270 liters/hr and nitrogen at 640 liters/hr were charged through a vaporizer and a preheater, as a downward flow. They were reacted at a reaction temperature of 620°C for one day. After cooling the reaction gas, reaction products were separated and collected in an absorption tower which employed water as its absorbent. In this case, the pressure loss of catalyst layers was 0.28 kg/cm$^2$.

The results on the first day after the start of the reaction were that the conversion of ethylene glycol was 99.8%, the selectivity of glyoxal was 54.6%, the selectivity of formaldehyde was 10.4%, and the selectivity of glycolaldehyde was 0.28%. A space-time yield was 2.3 g-GX/cm$^3$-cat•hr.

Furthermore, the feed of nitrogen was controlled to maintain the temperature at 620°C, and 30 days after the start of the reaction, the feed of nitrogen was 620 liters/hr, and the pressure loss of the catalyst layers was 0.27 kg/cm$^2$.

The results of the reaction were that the conversion of ethylene glycol was 99.6%, the selectivity of glyoxal was 55.1%, the selectivity of formaldehyde was 10.5%, and the selectivity of glycolaldehyde was 0.26%. The pressure loss of the catalyst layers and the results of the reaction scarcely changed from the initial results.

Example 4

$\alpha$-Alumina having a specific surface area of 1.3 m$^2$/g and a particle size distribution of 10 wt% of 405-150 $\mu$m, 26 wt% of 150-105 $\mu$m, 35 wt% of 105-75 $\mu$m and 29 wt% of 75-44 $\mu$m was treated by the same procedure as in Example 1 to obtain a catalyst containing 75 wt% of supported silver. Next, the obtained supported silver catalyst was classified, and a stainless steel reactor having an inner diameter of 27.4 mm was then filled with 10 ml of the obtained catalyst having particle sizes of 500-355 $\mu$m as the lowermost layer, 5 ml of the catalyst having particle sizes of 355-250 $\mu$m as the second layer, 5 ml of the catalyst having particle sizes of 250-150 $\mu$m as the third layer and 5 ml of the catalyst having particle sizes of 150-105 $\mu$m as the uppermost layer.

Into the thus-prepared reactor, ethylene glycol at 140 g/hr added trimethyl phosphite at the amount of 2.0 ppm as phosphorus to the ethylene glycol, water at 140 g/hr, air at 255 liters/hr and nitrogen at 510 liters/hr were charged through a vaporizer and a preheater, as a downward flow. They were reacted at a reaction temperature of 590°C for one day. After cooling the reaction gas, reaction products were separated and collected in an absorption tower which employed water as its absorbent. In this case, the pressure loss of catalyst layers was 0.19 kg/cm$^2$.

The results on the first day after the start of the reaction were that the conversion of ethylene glycol was 99.8%, the selectivity of glyoxal was 61.4%, the selectivity of formaldehyde was 11.6%, and the selectivity of glycolaldehyde was 0.37%. A space-time yield was 3.2 g-GX/cm$^3$-cat•hr.

Ethylene glycol, water, air and trimethyl phosphite were added into the reactor at 140 g/hr, 140 g/hr, 255 liters/hr and 2.0 ppm in term of phosphorus to ethylene glycol, respectively, and these conditions were kept constant and the feed of nitrogen was controlled to maintain the reaction temperature at 590°C. Thirty days after the start of the reaction, the feed of nitrogen was 500 liters/hr, and the pressure loss of the catalyst layer was 0.21 kg/cm$^2$. Sixty days after the start of the reaction, the feed of nitrogen was 480 liters/hr and the pressure loss of the catalyst layers was 0.20 kg/cm$^2$. In addition, 90 days after the start of the reaction, the feed of nitrogen was 460 liters/hr and the pressure loss of the catalyst layers was 0.20 kg/cm$^2$. Judging from the fact that the feed of nitrogen for maintaining the reaction temperature at 590°C scarcely changed between 90 days later and the initial stage, it could be considered that the activity of the catalyst was kept up.

The results of the reaction were that the conversion of ethylene glycol was 99.7%, the selectivity of glyoxal was 59.1%, the selectivity of formaldehyde was 12.2%, and the selectivity of glycolaldehyde was 0.24%. The pressure loss of the catalyst layers and the results of the reaction scarcely changed from the initial results.

Comparative Example 2

The same alumina as used in Example 4 was subjected to the same impregnation, drying and heat treatment as in Example 4 to obtain a catalyst containing 29 wt% of supported silver. Next, the obtained supported silver catalyst was classified, and a stainless steel reactor having an inner diameter of 27.4 mm was then filled with 10 ml of the obtained catalyst having particle sizes of 355-250 $\mu$m as the lowermost layer, 10 ml of the catalyst having particle sizes of 250-150 $\mu$m as the middle layer and 20 ml of the catalyst having particle sizes of 150-105 $\mu$m as the uppermost layer.

Into the thus-prepared reactor, ethylene glycol at 20 g/hr added trimethyl phosphite at the amount of 2.0 ppm as phosphorus to the ethylene glycol, water at 20 g/hr, air at 40 liters/hr and nitrogen at 180 liters/hr were charged through a vaporizer and a preheater, as a downward flow. They were reacted at a reaction temperature of 580°C for one day. After cooling the reaction gas, reaction products were separated and collected in an absorption tower which employed water as its absorbent.

In this case, the pressure loss of catalyst layers was 0.10 kg/cm$^2$.

The results on the first day after the start of the reaction were that the conversion of ethylene glycol was 97.9%, the selectivity of glyoxal was 70.3%, the selectivity of formaldehyde was 9.4%, and the selectivity of glycolaldehyde was 0.75%. A space-time yield was 0.32 g-GX/cm$^3$-cat•hr.

Next, ethylene glycol at 100 g/hr, water at 100 g/hr, air at 200 liters/hr, nitrogen at 600 liters/hr and trimethyl phosphite at the amount of 2.0 ppm in term of phosphorus to ethylene glycol were fed to the reactor as a downward flow. They were reacted at a temperature of 580°C. After cooling the reaction gas, reaction products were separated and collected in an absorption tower which employed water as its absorbent. The pressure loss of catalyst layers was 0.28 kg/cm$^2$.

The results of the reaction were that the conversion of ethylene glycol was 93.6%, the selectivity of glyoxal was 55.3%, the selectivity of formaldehyde was 12.4%, and the selectivity of glycolaldehyde was 1.83%. A space-time yield was 1.2 g-GX/cm$^3$-cat•hr.

Example 5

Silicon carbide having a specific surface area of 0.2 m$^2$/g and a particle size distribution of 10 wt% of 1,000-500 $\mu$m, 24 wt% of 500-250 $\mu$m, 29 wt% of 250-150 $\mu$m, 25 wt% of 150-105 $\mu$m and residual 12 wt% of 105-75 $\mu$m was treated by repeating the same impregnation, drying, heat treatment and milling as in Example 1 several times to obtain a catalyst containing 61 wt% of supported silver. Next, the obtained supported silver catalyst was classified, and a stainless steel reactor having an inner diameter of 27.4 mm was then filled with 5 ml of the obtained catalyst having particle sizes of 500-250 $\mu$m as the lowermost layer, 5 ml of the catalyst having particle sizes of 250-150 $\mu$m as the middle layer and 5 ml of the catalyst having particle sizes of 150-105 $\mu$m as the uppermost layer.

Into the thus-prepared reactor, ethylene glycol at 150 g/hr added trimethyl phosphite at the amount of 2.5 ppm as phosphorus to the ethylene glycol, water at 150 g/hr, air at 280 liters/hr and nitrogen at 600 liters/hr were charged through a vaporizer and a preheater, as a downward flow. They were reacted at a reaction temperature of 570°C for one day. After cooling the reaction gas, reaction products were separated and collected in an absorption tower which employed water as its absorbent.

The results on the first day after the start of the reaction were that the conversion of ethylene glycol was 99.8%, the selectivity of glyoxal was 62.1%, the selectivity of formaldehyde was 10.1%, and the selectivity of glycolaldehyde was 0.35%. A space-time yield was 5.8 g-GX/cm$^3$-cat•hr. In this case, the pressure loss of catalyst layers was 0.24 kg/cm$^2$.

Even after the reaction was further continued for 30 days, the pressure loss of the catalyst layers (0.25 kg/cm$^2$), the conversion of ethylene glycol (99.8%) and the selectivity of glyoxal (61.8%) scarcely changed.

Comparative Example 3

The same silicon carbide having a particle size of 500-250 $\mu$m as used in Example 5 was repeatedly subjected to the same impregnation, drying, heat treatment and milling as in Example 1 to obtain a catalyst containing 10 wt% of supported silver. Next, a stainless steel reactor having an inner diameter of 27.4 mm was then filled with 50 ml of the obtained catalyst having particle sizes of 500-250 $\mu$m.

Into the thus-prepared reactor, ethylene glycol at 10 g/hr added trimethyl phosphite at the amount of 1.0 ppm as phosphorus to the ethylene glycol, water at 12 g/hr, air at 25 liters/hr and nitrogen at 120 liters/hr were charged through a vaporizer and a preheater, as a downward flow. They were reacted at a reaction temperature of 500°C. After cooling the reaction gas, reaction products were separated and collected in an absorption tower which employed water as its absorbent. In this case, the pressure loss of catalyst layers was 0.09 kg/cm$^2$.

The results of the reaction were that the conversion of ethylene glycol was 92.9%, the selectivity of glyoxal was 75.6%, the selectivity of formaldehyde was 5.4%, and the selectivity of glycolaldehyde was 0.67%. A space-time yield was 0.16 g-GX/cm$^3$-cat•hr.

Next, the reactor was heated, and reaction was then carried out at a temperature of 600°C. After cooling the reaction gas, reaction products were separated and collected in the absorption tower. The pressure loss of catalyst layers was 0.11 kg/cm$^2$.

The results of the reaction were that the conversion of ethylene glycol was 98.2%, the selectivity of glyoxal was 45.3%, the selectivity of formaldehyde was 10.1%, and the selectivity of glycolaldehyde was 0.52%.

Example 6

For a silver alloy wire having a diameter of 0.2 mm and containing 7 wt% of palladium, thermal analysis (DTA-TG) was carried out. As a result, an endothermic peak began from about 1,020°C. This fact elucidated that the melting point of the alloy was 1,020°C.

This alloy wire of 200 m was cut into lengths of 5-10 mm, and a stainless steel reactor having an inner diameter of 27.4 mm was then filled with the cut alloy wire compressed to a height of 10 mm.

Into the thus-prepared reactor, ethylene glycol at 100 g/hr added trimethyl phosphite at the amount of 3.0 ppm as phosphorus to the ethylene glycol, water at 100 g/hr, air at 185 liters/hr and nitrogen at 400 liters/hr were charged through a vaporizer and a preheater, as a downward flow. They were reacted at a reaction temperature of 590°C. In this case, the pressure loss of catalyst layers was 0.14 kg/cm$^2$.

After cooling the reaction gas, reaction products were separated and collected in the absorption tower. The results of the reaction were that the conversion of ethylene glycol was 99.9%, the selectivity of glyoxal was 61.3%, the selectivity of formaldehyde was 10.2%, and the selectivity of glycolaldehyde was 0.21%. A space-time yield was 9.7 g-GX/cm$^3$-cat•hr.

Even after the reaction was further continued for 30 days, the pressure loss of the catalyst layers (0.14 kg/cm$^2$), the selectivity of glyoxal (61.4%) and the selectivity of glycolaldehyde scarcely changed.

Example 7

In a reactor, there were piled up 50 wire nets of 200 mesh which were made of a copper wire having a diameter of 0.053 mm and which were plated with silver as thick as 20 $\mu$m. In this case, the height of a filling layer was 20 mm.

Into the thus-prepared reactor, ethylene glycol at 136 g/hr added trimethyl phosphite at the amount of 3.0 ppm as phosphorus to the ethylene glycol, water at 136 g/hr, air at 285 liters/hr and nitrogen at 530 liters/hr were charged through a vaporizer and a preheater, as a downward flow. They were reacted at a reaction temperature of 560°C. In this case, the pressure loss of catalyst layers was 0.14 kg/cm$^2$.

After cooling the reaction gas, reaction products were separated and collected in the absorption tower. The results of the reaction were that the conversion of ethylene glycol was 99.6%, the selectivity of glyoxal was 62.1%, the selectivity of formaldehyde was 7.4%, and the selectivity of glycolaldehyde was 0.41%. A space-time yield was 6.7 g-GX/cm$^3$-cat•hr. Even after the reaction was further continued for 30 days, the pressure loss of the catalyst layers (0.15 kg/cm$^2$), the conversion of ethylene glycol (99.5%) and the selectivity of glyoxal (61.7%) scarcely changed.

Example 8

Zirconia having a specific surface area of 0.02 $m^2/g$ and a particle size distribution of 20 wt% of 250-150 $\mu$m, 31 wt% of 150-105 $\mu$m, 25 wt% of 105-75 $\mu$m and 24 wt% of 75-30 $\mu$m was chemically plated with Cu, and then electroplated with Ag as thick as 10 $\mu$m. In this case, the content of Ag was 56 wt%. Next, the obtained silver catalyst was classified, and a stainless steel reactor having an inner diameter of 27.4 mm was then filled with 10 ml of the obtained Ag-plated zirconia particles having particle sizes of 300-150 $\mu$m as the lower layer and 7 ml of the catalyst having particle sizes of 150-105 $\mu$m as the upper layer.

Into the thus-prepared reactor, ethylene glycol at 150 g/hr added triethyl phosphite at the amount of 1.0 ppm as phosphorus to the ethylene glycol, water at 150 g/hr, air at 280 liters/hr and nitrogen at 580 liters/hr were charged through a vaporizer and a preheater, as a downward flow. They were reacted at a reaction temperature of 590°C. After cooling the reaction gas, reaction products were separated and collected in the absorption tower. The results of the reaction were that the conversion of ethylene glycol was 99.5%, the selectivity of glyoxal was 55.1%, the selectivity of formaldehyde was 10.5%, and the selectivity of glycolaldehyde was 0.19%. In this case, the pressure loss of catalyst layers was 0.24 $kg/cm^2$ and a space-time yield was 4.5 g-GX/$cm^3$-cat•hr. Even after the reaction was further continued for 30 days, the pressure loss of the catalyst layers (0.19 $kg/cm^2$), the conversion of ethylene glycol (99.5%) and the selectivity of glyoxal (56.7%) and the selectivity of glycolaldehyde (0.20%) scarcely changed.

Example 9

Electrolyzed Cu particles having particle sizes of 150-44 $\mu$m were electroplated with Ag as thick as 15 $\mu$m. At this time, the content of Ag was about 58 wt%. A stainless steel reactor having an inner diameter of 27.4 mm was then filled with 20 ml of the obtained catalyst having particle sizes of 150-75 $\mu$m among the catalyst particles.

Into the thus-prepared reactor, ethylene glycol at 100 g/hr added triethyl phosphite at the amount of 1.5 ppm as phosphorus to the ethylene glycol, water at 100 g/hr, air at 200 liters/hr and nitrogen at 400 liters/hr were charged through a vaporizer and a preheater, as a downward flow. They were reacted at a reaction temperature of 595°C. The pressure loss of the catalyst layer was 0.25 $kg/cm^2$. After cooling the reaction gas, reaction products were separated and collected in the absorption tower. The results of the reaction were that the conversion of ethylene glycol was 99.8%, the selectivity of glyoxal was 57.2%, the selectivity of formaldehyde was 11.2%, and the selectivity of glycolaldehyde was 0.25%. Even after the reaction was further continued for 30 days, the pressure loss of the catalyst layers (0.27 $kg/cm^2$), the conversion of ethylene glycol (99.8%) and the selectivity of glyoxal (56.7%) scarcely changed.

In the above examples, it was intended to prepare the catalyst on a beaker scale, and so plastic and glass vessels were used. In the subsequent examples, the preparation of catalysts on a large scale will be described.

Example 10

Ten kilograms of silica having a specific surface area of 0.15 $m^2/g$ and a particle size distribution of 26 wt% of 400-150 $\mu$m, 35 wt% of 150-105 $\mu$m, 29 wt% of 105-75 $\mu$m, and 10 wt% of 75-35 $\mu$m were impregnated with an aqueous silver nitrate solution (which was prepared from 5 kg of silver nitrate and 2.5 kg of water), and the silica particles were then dried at 95°C for 12 hours, while stirred and mixed by a kneader (in which a reactor and stirring blades were both made of stainless steel). Next, the thus dried particles were placed in a ceramic crucible, thermally heated at 600°C for 30 minutes in an electrical muffle furnace in the atmosphere, cooled, crushed by a granulator (in which a crushing net and stirring blades were both made of stainless steel), and then classified. Furthermore, the operations of the same impregnation, drying, heat treatment, crushing and classification were repeated several times to prepare a catalyst containing 55 wt% of supported silver and having a specific surface area and a particle size distribution close to those of the raw material. Afterward, 10 g of the thus obtained catalyst were immersed into 100 ml of 4N-HCl overnight. The resultant supernatant liquid was analyzed, and as a result, impurities in the catalyst were 75 ppm of Fe, 16 ppm of Ni and 11 ppm of Cr.

Next, a stainless steel reactor having an inner diameter of 27.4 mm was then filled with 10 ml of the obtained catalyst having particle sizes of 400-250 $\mu$m as the lowermost layer, 10 ml of the catalyst having particle sizes of 250-150 $\mu$m as the second layer, 5 ml of the catalyst having particle sizes of 150-105 $\mu$m as the third layer and 5 ml of the catalyst having particle sizes of 105-75 $\mu$m as the uppermost layer.

Into the thus-prepared reactor, ethylene glycol at 100 g/hr added triethyl phosphite at the amount of 1 ppm as phosphorus to the ethylene glycol, water at 100 g/hr, air at 180 liters/hr and nitrogen at 450 liters/hr were charged through a vaporizer and a preheater, as a downward flow. They were reacted at a reaction temperature of 600°C for one day. The pressure loss of the catalyst layers was 0.31 kg/cm$^2$. After cooling the reaction gas, reaction products were separated and collected in an absorption tower which employed water as its absorbent.

The results on the first day after the start of the reaction were that the conversion of ethylene glycol was 98.5%, the selectivity of glyoxal was 54.2%, the selectivity of formaldehyde was 12.4%, and the selectivity of glycolaldehyde was 1.12%. A space-time yield was 1.7 g-GX/cm$^3$-cat•hr.

Example 11

A catalyst prepared in Example 10 was immersed into 4N-HCl for 6 hours, and then washed with water. Furthermore, it was immersed in 4N-NH$_3$ for 6 hours, washed with water, and then dried. Impurities in the catalyst were 2 ppm of Fe, 0.1 ppm or less of Ni and 0.1 ppm or less of Cr.

Next, a stainless steel reactor having an inner diameter of 27.4 mm was then filled with 10 ml of the thus treated catalyst having particle sizes of 400-250 μm as the lowermost layer, 10 ml of the catalyst having particle sizes of 250-150 μm as the second layer, 5 ml of the catalyst having particle sizes of 150-105 μm as the third layer and 5 ml of the catalyst having particle sizes of 105-75 μm as the uppermost layer.

Into the thus-prepared reactor, ethylene glycol at 100 g/hr added triethyl phosphite at the amount of 1 ppm as phosphorus to the ethylene glycol, water at 100 g/hr, air at 180 liters/hr and nitrogen at 550 liters/hr were charged through a vaporizer and a preheater, as a downward flow. They were reacted at a reaction temperature of 600°C for one day. The pressure loss of the catalyst layers was 0.32 kg/cm$^2$. After cooling the reaction gas, reaction products were separated and collected in an absorption tower which employed water as its absorbent.

The results of the reaction were that the conversion of ethylene glycol was 99.8%, the selectivity of glyoxal was 55.4%, the selectivity of formaldehyde was 11.7%, and the selectivity of glycolaldehyde was 0.26%. A space-time yield was 1.7 g-GX/cm$^3$-cat•hr.

Example 12

Silica spheres (diameter = 0.3 mm) having a specific surface area of 18 m$^2$/g were impregnated with a silver-ammonia complex ion solution (bis-amminesilver(I) acetate solution), mixed, and then dried at 80°C with stirring. Furthermore, the same impregnation and drying treatment were repeated several times, and the particles were then calcined at 600°C to prepare a catalyst containing 63 wt% of supported silver. Next, a reactor having an inner diameter of 27.4 mm was filled with 40 ml of the obtained supported silver catalyst.

Into the thus-prepared reactor, ethylene glycol at 100 g/hr, water at 100 g/hr, air at 200 liters/hr and nitrogen at 430 liters/hr were charged through a vaporizer and a preheater, as a downward flow. They were reacted at a reac tion temperature of 620°C for one day. After cooling the reaction gas, reaction products were separated and collected in an absorption tower which employed water as its absorbent. The pressure loss of the catalyst layers was 0.09 kg/cm$^2$.

The results on the first day after the start of the reaction were that the conversion of ethylene glycol was 99.0%, the selectivity of glyoxal was 39.8%, the selectivity of formaldehyde was 7.2%, and the selectivity of glycolaldehyde was 0.48%.

Moreover, on the second day after the start of the reaction, trimethyl phosphite was added to the reactor at the amount of 2.0 ppm in term of phosphorus to ethylene glycol, and ethylene glycol, water, air and nitrogen were further fed thereto at 100 g/hr, 100 g/hr, 200 l/hr and 410 l/hr, respectively, as a downward flow. Reaction was then carried out at a temperature of 605°C. The pressure loss of the catalyst layers was 0.09 kg/cm$^2$.

The results of this reaction were that the conversion of ethylene glycol was 98.7%, the selectivity of glyoxal was 51.7%, the selectivity of formaldehyde was 6.6%, and the selectivity of glycolaldehyde was 0.51%. A space-time yield was 1.2 g-GX/cm$^3$-cat•hr.

The reaction was continued, while the feeds of ethylene glycol, water, air and trimethyl phosphite were constantly kept up and the feed of nitrogen was controlled so that the reaction temperature might be maintained at 605°C.

Thirty dyes after the start of the reaction, the feed of nitrogen necessary to maintain the reaction temperature at 65°C was 410 l/hr, which was the same as the feed of nitrogen on the second day. Thus, it could be considered that the activity of the catalyst did not change even after 30 days.

Thirty days after the start of the reaction, the pressure loss of the catalyst layer was 0.09 kg/cm$^2$. The results of this reaction were that the conversion of ethylene glycol was 98.9%, the selectivity of glyoxal was 52.1%, the selectivity of formaldehyde was 6.7%, and the selectivity of glycolaldehyde was 0.47%.

**Claims**

1. A method for preparing glyoxal which comprises oxidizing/dehydrogenating ethylene glycol at 500-700°C in the presence of a silver catalyst in which at least 50 wt% of silver is supported on a carrier.

2. The method for preparing glyoxal according to Claim 1 wherein the oxidizing/dehydrogenating reaction is carried out in the presence of a phosphorus compound.

3. The method for preparing glyoxal according to Claim 1 wherein said carrier is an oxide, a nitride, a car bide or its intermediate composition.

4. The method for preparing glyoxal according to Claim 1 wherein said carrier is particles of a simple metal or an alloy.

5. The method for preparing glyoxal according to Claim 1 wherein said carrier is particles of an inert inorganic compound.

6. The method for preparing glyoxal according to Claim 1 wherein said carrier is a wire or a wire net of a simple metal or an alloy.

7. The method for preparing glyoxal according to Claim 1 wherein said silver catalyst is obtained by supporting at least 50 wt% of silver on the carrier, and then im mersing the supported silver catalyst into an acid which does not react with silver.

8. The method for preparing glyoxal according to Claim 2 wherein the amount of said phosphorus compound to be added is in the range of from 0.01 to 10.0 ppm in term of phosphorus to ethylene glycol.

9. The method for preparing glyoxal according to Claim 3 wherein said oxide is silica or $\alpha$-alumina.

10. The method for preparing glyoxal according to Claim 3 wherein said oxide, nitride, carbide or intermediate composition has a specific surface area of 10.0 m$^2$/g or less, a particle size of substantially 10 to 500 $\mu$m, and a dielectric constant of 25 or more.

11. The method for preparing glyoxal according to Claim 3 wherein said silver catalyst is obtained by impregnating the particles of said oxide, nitride, carbide or intermediate composition with a silver salt solution, followed by a heat treatment to cause thermal decomposition, to support silver on the particles.

12. The method for preparing glyoxal according to Claim 5 wherein said silver catalyst is supported on the particles of the inert inorganic compound by plating.

13. The method for preparing glyoxal according to Claim 6 wherein said silver catalyst is supported on the wire or wire net of the simple metal or the alloy by plating.

14. The method for preparing glyoxal according to Claim 9 wherein said silica has a specific surface area of 5.0 m$^2$/g or less and a particle size of substantially 10 to 1,000 $\mu$m.

15. The method for preparing glyoxal according to Claim 9 wherein said $\alpha$-alumina has a specific surface area of 5.0 m$^2$/g or less and a particle size of substantially 10 to 500 $\mu$m.

16. A method for preparing glyoxal which comprises oxidizing/dehydrogenating ethylene glycol at 500-700°C in the presence of a catalyst comprising a silver alloy having a melting point higher than the melting point of silver.

17. The method for preparing glyoxal according to Claim 16 wherein the oxidizing/dehydrogenating reaction is carried out in the presence of a phosphorus compound.

18. The method for preparing glyoxal according to Claim 17 wherein the amount of said phosphorus compound to be added is in the range of from 0.01 to 10.0 ppm in term of phosphorus to ethylene glycol.

19. The method for preparing glyoxal according to Claim 1 wherein ethylene glycol is oxidized/dehydrogenated at 500-700°C again in the presence of a catalyst activated by immersing said silver catalyst used in the oxidizing/dehydrogenating reaction of ethylene glycol into an acid which does not react with silver.

20. A silver catalyst for the preparation of glyoxal in which at least 50 wt% of silver is supported on a carrier.

21. The silver catalyst according to Claim 20 wherein said carrier is an oxide, a nitride, a carbide or its intermediate composition.

22. The silver catalyst according to Claim 20 wherein said carrier is particles of a simple metal or an alloy.

**23.** The silver catalyst according to Claim 20 wherein said carrier is particles of an inert inorganic compound.

**24.** The silver catalyst according to Claim 20 wherein said carrier is a wire or a wire net of a simple metal or an alloy.

**25.** The silver catalyst according to Claim 20 wherein said silver catalyst is obtained by supporting at least 50 wt% of silver on the carrier, and then immersing the supported silver catalyst into an acid which does not react with silver.

**26.** The silver catalyst according to Claim 21 wherein said oxide is silica or $\alpha$-alumina.

**27.** The silver catalyst according to Claim 21 wherein said oxide, nitride, carbide or intermediate composition has a specific surface area of 10.0 m$^2$/g or less, a particle size of substantially 10 to 500 $\mu$m, and a dielectric constant of 25 or more.

**28.** The silver catalyst according to Claim 21 wherein said silver catalyst is obtained by impregnating the particles of said oxide, nitride, carbide or intermediate composition with a silver salt solution, followed by a heat treatment to cause thermal decomposition, to support silver on the particles.

**29.** The silver catalyst according to Claim 23 wherein said silver catalyst is supported on the particles of the inert inorganic compound by plating.

**30.** The silver catalyst according to Claim 24 wherein said silver catalyst is supported on the wire or wire net of the simple metal or the alloy by plating.

**31.** The silver catalyst according to Claim 26 wherein said silica has a specific surface area of 5.0 m$^2$/g or less and a particle size of substantially 10 to 1,000 $\mu$m.

**32.** The silver catalyst according to Claim 26 wherein said $\alpha$-alumina has a specific surface area of 5.0 m$^2$/g or less and a particle size of substantially 10 to 500 $\mu$m.

**33.** A silver catalyst for the preparation of glyoxal which comprises a silver alloy having a melting point higher than the melting point of silver.

**34.** The silver catalyst according to Claim 20 which is activated by immersing said silver catalyst used in the oxidizing/dehydrogenating reaction of ethylene glycol into an acid which does not react with silver.

**35.** The silver catalyst according to Claim 20 which is obtained by recovering said silver catalyst used in the oxidizing/dehydrogenating reaction of ethylene glycol, and then crushing the same.

**36.** A method for activating a silver catalyst for the preparation of glyoxal which comprises the step of immersing a silver catalyst used in the oxidizing/dehydrogenating reaction of ethylene glycol into an acid which does not react with silver to activate said silver catalyst.

**37.** A method for activating a silver catalyst for the preparation of glyoxal which comprises the steps of supporting at least 50 wt% of silver on a carrier, and then immersing the supported silver catalyst into an acid which does not react with silver.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | DE-A-38 37 103 (HOECHST AG)<br><br>* page 3, line 10 - line 28; claims * | 1-3,<br>9-11,14,<br>15,20,<br>21,<br>26-28,<br>31,32 | C07C45/38<br>C07C47/127<br>B01J23/50 |
| A | DATABASE WPI<br>Week 9148,<br>Derwent Publications Ltd., London, GB;<br>AN 91-349186<br>& JP-A-3 232 835 (MITSUI TOATSU CHEM INC)<br>16 October 1991<br>* abstract * | 1-3,8,<br>10,11 | |
| A | US-A-4 359 587 (M. ABDURAKHMANOV ET AL.)<br>* column 7; claims * | 1,3,9-11 | |
| A | CHEMICAL ABSTRACTS, vol. 56, no. 4,<br>1962, Columbus, Ohio, US;<br>abstract no. 3359d,<br>M.S. BRODSKII ET AL. 'Glyoxal'<br>column 3359 ;<br>* abstract *<br>& SU-A-136 352 (M.S. BRODSKII ET AL.) 14<br>March 1961 | 1,3,9 | TECHNICAL FIELDS SEARCHED (Int.Cl.5)<br><br>C07C |
| A | CHEMICAL ABSTRACTS, vol. 62, no. 1,<br>1965, Columbus, Ohio, US;<br>abstract no. 447c,<br>M.S. BRODSKII ET AL. 'Preparation of<br>glyoxal'<br>column 447 ;<br>* abstract *<br>& SU-A-164 262 (SCIENTIFIC-RESEARCH<br>INSTITUTE OF ORGANIC INTERMEDIATES AND<br>DYES) 13 August 1964 | 1 | |

-/--

The present search report has been drawn up for all claims

| Place of search | Date of completion of the search | Examiner |
|---|---|---|
| THE HAGUE | 2 December 1993 | Bonnevalle, E |

EPO FORM 1503 03.82 (P04C01)

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | DE-A-39 04 829 (NIPPON GOHSEI KAGAKU KOGYO K.K.) <br> * column 3; claims * <br> ----- | 1 | |
| | | | TECHNICAL FIELDS SEARCHED (Int.Cl.5) |

The present search report has been drawn up for all claims

| Place of search | Date of completion of the search | Examiner |
|---|---|---|
| THE HAGUE | 2 December 1993 | Bonnevalle, E |

EPO FORM 1503 03.82 (P04C01)